# EUROPEAN PATENT SPECIFICATION

(11) **EP 0 371 692 B1**
(45) Date of publication and mention of the grant of the patent: **25.09.1996**
(21) Application number: 89312156.6
(22) Date of filing: 22.11.1989
(51) Int. Cl.: C07C 11/09, C07C 11/10, C07C 43/04, C07C 7/12, C07C 41/06

(54) **Verfahren zur Herstellung eines Ethers.**
Process for the production of an ether.
Procédé pour la production d'un éther.

(30) Priority: 22.11.1988 US 274557
(43) Date of publication of application: 06.06.1990
(73) Proprietor: EXXON CHEMICAL PATENTS INC., Florham Park New Jersey 07932 (US)
(72) Inventor: Cikut, John Joseph, Kingwood Texas 77339-3640 (US); Michaelson, Robert Charles, Kinnelon New Jersey 07405 (US); Hendriksen, Dan Eldon, Kingwood Texas 77345 (US); Fons, Terry Allen, Baton Rouge, LA 70817 (US); Ou, Di-yu Nmn, Houston Texas 77062 (US); Rosenfeld, Daniel David, Houston Texas 77059 (US)
(74) Representative: Bawden, Peter Charles

(56) References cited:
- EP-A- 0 016 284
- EP-A- 0 047 906
- EP-A- 0 071 238
- EP-A- 0 129 842
- GB-A- 1 228 094
- US-A- 2 862 856
- US-A- 3 760 029
- US-A- 4 504 687
- US-A- 4 540 842

## Description

### 1. Field of the Invention

The present invention relates to methods of conducting catalytic chemical reactions under conditions so as to minimize or substantially avoid deactivation of the catalyst material due to a reaction of dialkyl sulfides with the catalyst material. More particularly, the present invention relates to the preparation of alkyl tertiary alkyl ether by catalytic reaction of hydrocarbon streams containing mixtures of isoolefins and alcohols under conditions which minimize or substantially avoid reaction of the catalytic material in the catalytic reaction zone with any dialkyl sulfides which may be present in the stream. Specifically, the present invention it directed to a catalytic reaction for producing alkyl tertiary alkyl ether which involves removing dialkyl sulfides from the hydrocarbon stream prior to introduction of the stream into the catalytic reaction zone and, optionally, introducing sufficient amounts of oxygenates to the reaction zone to inhibit reaction of dialkyl sulfides which may be present in the stream with the catalyst material.

### 2. Discussion of Background and Material Information

Methyl tertiary butyl ether (MTBE) in recent years has become an important product of the petroleum industry in view of its acceptance as a non-environmentally harmful octane improver for gasolines.

In view of the octane improving characteristics of MTBE, processes, such as those disclosed in U.S. Patent Nos. 3,726,942 and 3,846,008, have been developed to separate isoolefins, and isobutene in particular, from mixtures with corresponding normal olefins and alkanes which involves etherifying the isoolefins with a C₁ to C₆ primary alcohol in the presence of an acidic cation exchange resin catalyst followed by fractionation to separate the low boiling hydrocarbons from the higher boiling ether.

In a variation of these processes, as described in U.S. Patent Nos. 3,629,578 and 3,634,534, HAUNSCHILD, the mixture of isoolefin and normal olefin with lower primary alcohols is fed to a distillation column in which there are a plurality of zones of acidic ion exchanges resin catalysts whereby the isoolefin ether is formed and drops to the bottom of the column while normal olefins, and paraffins, are distilled overhead.

More recently, catalytic distillation processes, such as those disclosed in U.S. Patent Nos. 4,232,177, 4,307,254, and 4,336,407, SMITH, Jr., have been developed to improve the recovery of MTBE.

EP-A-47906 discloses a process in which an isoolefin and an alcohol are reacted in the presence of an acid ion exchange catalyst to form an ether. The isoolefin is pretreated before being passed over the catalyst with an acid to reduce the amount of basic materials (generally ammonia) present.

EP-A-16284 discloses a process for producing a substantially sulphur-free butene-1 rich stream from a butene-containing C₄ hydrocarbon feedstream which is suitable for rhodium-catalysed hydroformylation to produce n-valeraldehyde.

Notwithstanding the recent attempts to improve the production of isobutene and MTBE, a problem associated with conventional processes for the production of MTBE is that the catalyst material used in the catalyst reaction processes has a tendency to deactivate in an unacceptably short period of time.

### SUMMARY OF THE INVENTION

The present invention is the discovery that dialkyl sulfides, such as dimethyl sulfide, if present in hydrocarbon streams, react with acidic sites on catalyst material so as to result in the neutralization of these sites with the concomitant loss of catalyst activity.

According to the present invention there is provided a process for the production of an ether, such as an alkyl tertiary alkyl ether having a normal boiling point in the range of 54°C to 93°C (130°F-200°F), and particularly MTBE, by contacting a hydrocarbon feed stream in a reaction zone of a reactor in the presence of an acid catalyst characterised in that to minimise deactivation of said catalyst resulting from a reaction of dialkyl sulfide with said catalyst, dialkyl sulfide is removed from the hydrocarbon feed stream by adsorption prior to introduction into the reactor.

Preferably the adsorption of the dialkyl sulfides is performed in a cyclic operation involving the use of two adsorption columns.

In another embodiment, the catalytic reaction is performed by also introducing sufficient amounts of oxygenates to the reaction zone to inhibit the reaction of the dialkyl sulfides present in the feed with the catalyst material. In accordance with the present invention, the oxygenates suitable for this purpose are preferably oxygen-containing hydrocarbons, such as those selected from the group consisting of ethers, alcohols and mixtures thereof, preferably, wherein the ethers are selected from the group consisting of methyl sec-butyl ether, methyl n-butyl ether, and tert-amyl methyl ether (TAME), and most preferably TAME, and wherein the alcohol is selected from the group consisting of C₃ alcohols, C₄ alcohols, C₅ alcohols, ethanol and methanol, with methanol being preferred. In this case, the hydrocarbon stream is subjected to the adsorption technique to remove dialkyl sulfides from the stream prior to adding oxygenates into the stream for introduction into the catalytic reaction zone.

The diakly sulfide found in hydrocarbon streams which has been discovered to be the most responsible for deactivation of the catalyst is dimethyl sulfide.

For purposes of producing methyl tertiary butyl ether (MTBE), the preferred components of the feed comprise isobutene and methanol.

Catalysts which have been found to be suitable for use in this process of the present invention include cation exchange resins. Preferred catalysts for purposes of performing catalytic distillation processes in accordance with the present invention, however, are acid catalysts, such as acidic resin catalysts. A more preferred catalyst for purposes of the present invention is a macroreticular sulfonic acid cation exchange resin, with a member selected from the group consisting of Amberlyst 15, Lewatit SPC 118 BG (manufactured by Mobay/Bayer), Dowex M-31 and Dowex DR-2040 (manufactured by Dow Chemical Company) being more preferred, with Dowex DR-2040 being most preferred.

In a typical hydrocarbon stream subjected to a catalytic reaction process, i.e., one which has not been subjected to a treatment effective to reduce or substantially eliminate dialkyl sulfides from the stream in accordance with one embodiment of the present invention, dimethyl sulfide may be present in an amount up to about 4 wt%, and more typically in an amount within the range of up to about 10 ppm - 50ppm. In such case the oxygenate, such as methanol, is present in the catalyst zone in an amount of at least about 4% by weight of said hydrocarbon stream and preferably 4% to 7% in the liquid phase in the region of the distillation tower containing the catalyst.

### BRIEF DESCRIPTION OF THE DRAWING

The Figures annexed hereto are flow diagrams showing catalytic distillation processes in accordance with the present invention.

Fig. 1 is a flow diagram of a catalytic distillation process wherein oxygenates are introduced into the distillation column.

Fig. 2 shows a substantially identical flow diagram as in Fig. 1 except an adsorption column is installed prior to the mixing point of the hydrocarbon stream feed and the methanol stream.

### DETAILED DESCRIPTION

The present invention is based on the discovery that typical hydrocarbon streams which are subjected to catalytic reaction processes in the producing of ether, such as alkyl tertiary alkyl ethers, and particularly such ethers having a normal boiling point within the range of 54°C to 93°C (130°F-200°F) and most notably MTBE, contain dialkyl sulfides, such as dimethyl sulfide (DMS), which react in the presence of the acidic sites on the catalyst material so as to result in the neutralization of these sites with the concominant loss of catalyst activity. This has been found to be particularly the case for the production of MTBE by catalytic distillation reaction processes. Thus, the present invention relates to performing catalytic reactions in a manner which minimizes or substantially avoids reaction of dialkyl sulfides, and particularly dimethyl sulfide, which may be present in the hydrocarbon stream when fed or introduced into the catalytic reaction zone, such as a catalytic distillation column, even though the hydrocarbon stream may have previously been subjected to procedures in an attempt to remove contaminants, such as cationic material, therefrom which were believed to be responsible for deactivation of catalysts.

One embodiment of the present invention, relates to catalytic reaction processes for producing ethers from hydrocarbon streams containing dialkyl sulfides, and particularly catalytic reactions of isoolefins, such as isobutene, containing dimethyl sulfide, over an acid catalyst, such as an acid resin catalyst.

In this embodiment of the present invention, which may also be referred to herein as dimethyl sulfide adsorption, a hydrocarbon stream essentially devoid of dialkyl sulfides, such as dimethyl sulfide, is provided for example by removing, and preferably substantially eliminating all, dialkyl sulfide from the hydrocarbon stream prior to contacting the hydrocarbon stream with the acidic catalyst material in the catalytic reaction zone, preferably in the distillation column used in a catalytic distillation reaction process.

In a further embodiment, the deactivation of the catalyst material used in such catalytic reaction processes is minimized or substantially eliminated by also introducing oxygenates in addition to the hydrocarbon stream into the catalytic reaction zone after removing dialkyl sulfides from the hydrocarbon stream.

An embodiment related to the previously mentioned embodiments is a catalytic reaction process which involves reducing or substantially eliminating diakly sulfides, and preferably dimethyl sulfide, from the hydrocarbon stream, preferably by an adsorption technique, prior to the stage in the process where the hydrocarbon stream and oxygenates, such as methanol, are introduced into the catalytic reaction zone.

Another embodiment contemplated for purposes of the present invention is to provide a catalyst appropriate for the reaction of choice for the production of alkyl tertiary alkyl ethers, such as those having a normal boiling point in the range of 54°C to 93°C (130°F-200°F), e.g. MTBE, which is essentially non-susceptible to reaction with the diaklyl sulfides, such as dimethyl sulfide, present in the hydrocarbon feed.

Although the detailed description of the present invention including the preferred and best mode is specific to the production of methyl tertiary butyl ether wherein the hydrocarbon stream comprises isobutene and methanol, it should be understood that the present invention is also applicable to the production of other alkyl tertiary alkyl ethers, and particularly those having a normal boiling point falling within the range of 54°C to 93°C (130°F to 200°F), wherein the isoolefins and alcohols used would be selected from the groups of isoolefins and alcohols suitable for the reaction of choice in the production of the desired alkyl tertiary alkyl ether. For example in the case of the production of tertiary amyl methyl ether (TAME), the isoolefin may be an isoamylene such as either or both of 2-methy-butene-1 and 2-methy butene-2, and the alcohol is methanol. Ethanol, however, would be the alcohol used to produce tertiary amyl ethyl ether and ethyl tertiary butyl ether. Suitable alcohols useful for purposes of the present invention, therefore, include C₃ alcohols, C₄ alcohols, C₅ alcohols and ethanol in addition to methanol.

### The Oxygenate Procedure

This method of the present invention involves feeding a mixture containing isobutene and dimethyl sulfide into feed zone of a reactor, feeding methanol and/or other oxygenates into the feed zone, and contacting the resultant mixture of isobutene, dimethyl sulfide, and methanol with a fixed bed acidic cation exchange resin in the reaction zone thereby catalytically reacting the isobutene with the methanol under conditions which favor forming resultant MTBE while inhibiting reaction of the dimethyl sulfide with the catalyst material so as to substantially minimize or avoid deactivation of the catalyst material.

A critical parameter in the manufacture of MTBE is the maintenance of high catalytic activity. In the synthesis of MTBE, as practiced in the art, however, catalyst deactivation has been shown to occur by different mechanisms in different areas of the process. For example, in the fixed bed or tubular reactor, wherein an acidic resin, such as Amberlyst 15 (trademark), is employed to catalyze the formation of MTBE from isobutene and methanol, deactivation of the catalyst occurs over time if the catalyst is exposed to cationic or strongly basic material, such as metals, nitrogen compounds, and the like. In order to drive the reaction of methanol and isobutene to MTBE to completion, however, it has been proposed to use the same acidic resin catalyst downstream of a first stage reactor, thereby permitting more complete utilization of the isobutene in the feed.

Due to the relatively low concentration of oxygenates, such as methanol, in the downstream portion of the system, however, the catalyst has a tendency to deactivate over time even in the absence of cationic material. Prior to the discovery of the present invention, i.e., that this deactivation results from the reaction of low levels, i.e., as low as 10 ppm or lower, of dimethyl sulfide with highly acidic catalyst sites which are present primarily due to the relatively low levels of methanol, i.e., about 0.6-2 wt. %, and MTBE in the reaction zone, it is not believed that those skilled in the art had identified the cause of the problem, much less taught or suggested the solution for the problem which is the crux of the present invention as described in more detail hereinbelow.

One embodiment of the present invention, therefore, is the discovery that increasing the levels of oxygenates, i.e., methanol or other alcohols as well as ethers, attenuates the acidity of the catalyst so that reaction between dimethyl sulfide and catalyst is substantially reduced without adversely affecting the reaction of choice, i.e., the reaction of alcohol and isobutene to MTBE.

Where oxygenates, such as methanol, are introduced into the reaction zone, the catalyst material may be any acid catalyst appropriate for the reaction, such as catalytic metals and their oxides or halides suitable for a multitude of catalytic reactions and particularly heterogeneous with the reaction or other fluids in the system.

For example, where the present invention is practiced in a catalytic distillation process, the catalytic material may be in any form which permits its incorporation into a distillation tower, such as a fixed bed, but may also be in a form which serves as a distillation packing, for example, rings, saddles, balls, irregular pieces, sheets, tubes, spirals, packed in bags, plated on grills or screens, and reticulated polymer foams.

Catalysts which have been found to be suitable for use in this process of the present invention include cation exchange resins. Preferred catalysts for purposes of the present invention, however, are acid catalysts, such as acidic resin catalysts. A more preferred catalyst for purposes of the present invention is a macroreticular sulfonic acid cation exchange resin, selected from the group consisting of Amberlyst 15 (trademark), Lewatit SPC 18 BG, Dowex M-31, and Dowex DR-2040, with Dowex DR-2040 being most preferred.

In accordance this embodiment of the present invention, it has been found that deactivation of the acidic resin catalyst can be substantially reduced by maintaining the methanol concentration in the liquid phase in the catalyst reaction zone preferably at about 4 wt. % or higher. As an alternative, catalyst deactivation can be substantially reduced by injecting or recycling oxygenates, such as any suitable oxygen-containing hydrocarbon, e.g., alcohols or ethers, into the catalyst zone at concentrations appropriate for the particular oxygenate. For example, compounds which normally co-exist in the feedstreams from which isobutene is normally reacted, but in low concentrations, are suitable for this purpose. These include, but are not limited to, methyl sec-butyl ether, methyl n-butyl ether, tert-butyl alcohol, C₃ alcohols, C₅ alcohols, ethanol, methanol, methyl tert butyl ether (MTBE), and tert-amyl methyl ether (TAME). Of these, TAME is preferred in that it imparts several distinct advantages because its presence does not impede the formation of MTBE and because it is easily separable from the reaction products and recycled.

### EXAMPLE I

The following tests were conducted as evidence that dimethyl sulfide, and not other sulfur-containing compounds, is a poison for Amberlyst-15 (trademark) acid catalyst under conditions used commercially in the production of MTBE.

The reactions were carried out in a laboratory-scale, continuous-flow tubular reactor. The acid catalyst resin, generally 10 cc, was placed in a metal tube and held in place by glass wool plugs. The tube was jacketed by a larger tube with circulating hot water to control the temperature of the reaction, which was generally held at 70°C. The single liquid feed was introduced by a metering pump controlling the flow of the liquid to yield a Liquid Hourly Space Velocity (LHSV) of 4. The back pressure at the exit of the reactor, 1241 kPa, (180 psig), was kept high enough so that the reactor was filled by liquid, with no vapor. The acid catalyst used for purposes of this test was Amberlyst-15 (trademark), obtained as a fresh sample from a commercial plant. The feed to the laboratory reactor was also obtained from a commercial plant, and was composed of 2.67 wt.% isobutylene, and more than enough methanol to react with the isobutylene to yield in MTBE, with the balance of the feed being essentially mixed butenes and butanes. The progress of the reaction was monitored by taking small liquid samples under pressure from the reactor exit and analyzing them on a capillary gas chromatograph. The conversion of isobutylene was then calculated and was used to monitor the activity of the catalyst.

For purposes of this comparison, the following tests were run:

**TABLE I**

| Run | Feed Additive | Observations |
|---|---|---|
| IA | no additive | Isobutylene conversion remained constant at 90% for 16 hours. |
| IB | 5.6 wt.% Dimethyl Sulfide | The isobutylene conversion declined steadily from 90% to less than 50% over 19 hours. The spent catalyst in this reaction analyzed for in acidity of 2.8 milliequivalents per gram, down from the normal 4.5 meq/g in fresh catalyst. |
| IC | 5.6 wt.% Ethyl mercaptan | The isobutylene conversion remained constant it 90% for 11 hours; this was followed by straight feed for 18 hours, with isobutylene conversion again remaining constant at 90%. |
| ID | 2.9 wt.% Dimethyl sulfide 3.0 wt.% Ethyl mercaptan | The isobutylene conversion declined steadily over 18 hours at half the rate as when 5.6 wt.% dimethyl sulfide was added, for example in Run IB. |
| IE | 7.3 wt.% Methyl t-butyl sulfide | The isobutylene conversion remained constant at 88% for 17 hours, followed by straight feed for 7 hours, with the isobutylene conversion remaining constant at 89%. |

The foregoing comparison demonstrates that under these conditions, dimethyl sulfide is a poison for the acid catalyst used to produce MTBE from isobutylene and methanol, and that the tendency of dimethyl sulfide to poison the catalyst is not affected by the presence of other sulfur-containing compounds, such as mercaptan, e.g., ethyl mercaptan, nor is the catalyst poisoned by other organosulfur compounds, such as ethyl mercaptan or methyl tertiary-butyl sulfide.

### EXAMPLE II (Comparative)

The following comparison demonstrates the effect of the methanol content of the feed on the dimethyl sulfide poisoning of the catalyst. The run conditions used were substantially the same as those used in the previous Example. For purposes of this Example, the feed had an initial methanol content of about 3 wt.% and an isobutylene content of about 3.2 wt.%. For Runs IIA and IIB the methanol concentration was adjusted to 4%. In Run IIA, 0.5 wt.% dimethyl sulfide was introduced into the feed; and for Run IIB, 2 wt.% dimethyl sulfide was included in the feed. In Run IIC, 5 wt.% dimethyl sulfide was included in the feed and an additional 3 wt.% methanol for a total of 6 wt.% methanol was included in the feed. In Run IID, the methanol content of the feed was 1.5 wt.% and isobutylene content was 2.5 wt.%; and 5 wt.% of dimethyl sulfide was added to the feed.

The results of the observations are tabulated below:

**TABLE II**

| Run | Dimethyl Sulfide wt.% | Methanol wt.% | Observations |
|---|---|---|---|
| IIA | 0.5 | 4.0 | The conversion remained at 85-90% and the performance of the catalyst was not affected over a period of a 15 to 18 hour run. |
| IIB | 2.0 | 4.0 | The conversion remained at 85-90%, and the performance of the catalyst was not affected over a period of a 15 to 18 hour run. |
| IIC | 5.0 | 6.0 | Very little effect manifested by a drop to about 75% conversion after 15 hours of the run. |
| IID | 5.0 | 1.5 | Resulted in poisoning of the catalyst in a period of 8 to 12 hours of the run during which the conversion dropped to less than 50%. |

The foregoing comparison demonstrates that feeds containing about 4 wt.% methanol spiked with 0.5 to 2 wt.% dimethyl sulfide do not poison the catalyst over a run period of 15 to 18 hours. Similarly, feeds containing about 6 wt.% methanol spiked with 5 wt.% dimethyl sulfide appear to experience a small reduction of the conversion after 15 hours of a run. In contrast, a feed containing about 1.5 wt.% methanol and 5 wt.% dimethyl sulfide, however, poisons the catalyst within 8 to 12 hours of the run.

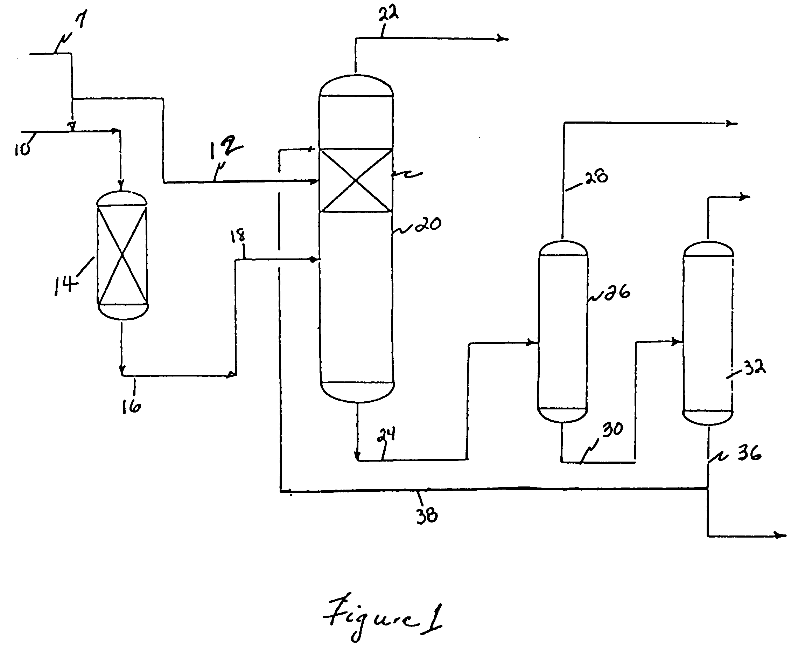

Referring now to Figure 1, a schematic system is shown, which can be used to produce MTBE.

A feed stream 7 containing a stoichiometric amount of methanol based on isobutylene is introduced together with an isobutylene containing feed steam 10 to a lead synthesis reactor 14. The lead synthesis reactor 14 is provided with an acidic resin catalyst, such as Amberlyst-15 (trademark), Dowex Dr-2040, Lewatit SPC 18 BG, or Dowex M-31, and is heated to an appropriate temperature. The effluent or product stream 16 leaving the reactor is composed of MTBE, unreacted hydrocarbons and methanol (MeOH). The resultant product stream is the feedstream 18 which is then fed to a distillation column 20. The vaporized overhead 22 is composed of raffinate depleted in olefins branched at the point of unsaturation (sometimes referred to as tertiary olefins) which is passed through methanol removal and final clean-up procedures. In accordance with the present invention, however, a stream 12 of methanol is introduced into the catalystic distillation reaction zone, wherein the catalyst may also be Amberlyst 15 or equivalent but is preferably Dowex DR-2040. The effluent is then passed to a product topping tower 26 wherein C₅ hydrocarbons are removed for separate processing. The resultant effluent stream 30 is then passed to product tailing tower wherein MTBE is removed as product. The effluent 36 from tailing tower contains various components including oxygenates, such as TAME, which are recycled through conduit 38 to supply oxygenate the catalyst reaction zone.

A catalytic reaction process which is particularly amenable to being practiced in accordance with the present invention may be a catalytic process performed in a conventional manner, such as that which is disclosed in U.S. Patent Nos. 4,232,177, 4,307,254 and 4,336,401.

### Dimethyl Sulfide Adsorption

In accordance with the present invention, a procedure has been developed to minimize or substantially eliminate the deleterious effects which would otherwise be caused by the presence of dimethyl sulfide in the catalytic reaction zone. This procedure may be used in conjunction with the introduction of oxygenates to better ensure that the catalytic material is not subject to a reaction with dimethyl sulfide.

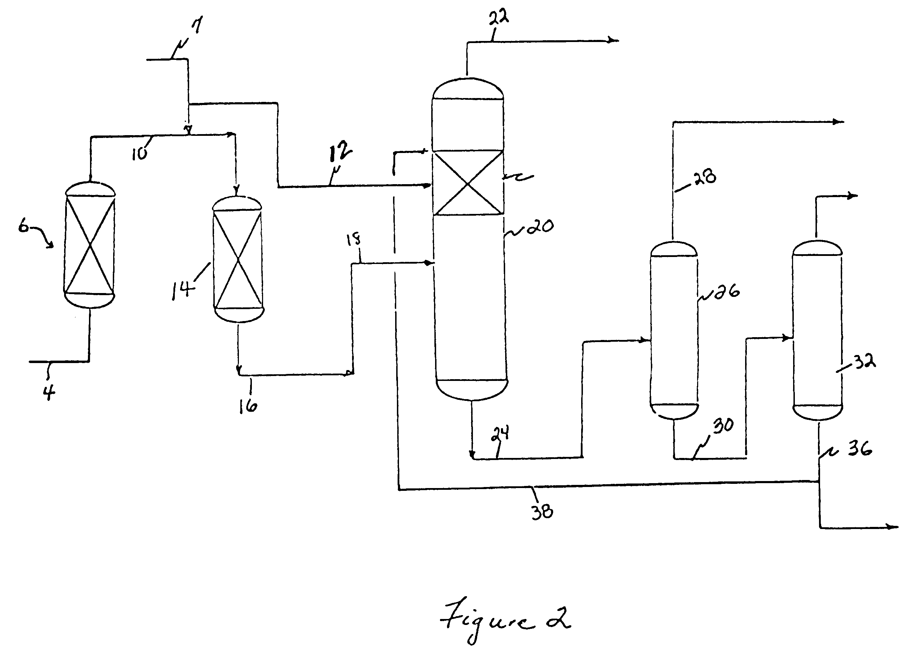

In this procedure, the sulfide contaminants in the hydrocarbon stream may be removed by installing an adsorption column prior to the mixing point of feed 4 and methanol 7 streams in a catalytic distillation procedure othervise the same as discussed above with respect to Figure 1. The removal has been discovered to be most effective for a methanol-free feed stream. This arrangement is illustrated in the Figure 2. In practice, the removal is preferably accomplished with a cyclic operation involving the use of two adsorption columns so that while one column is adsorbing the sulfides, the other column is being regenerated to recover the capacity.

The adsorbents suitable for removing dialkyl sulfides for purposes of the present invention include crystalline aluminosilicates, such as zeolite X, zeolite Y, zeolite Beta, silicalite, mordenite, and metal oxides, such as cobalt oxide, chromium oxide, nickel oxide and molybdenum oxide, supported on alumina and carbon.

### EXAMPLE III

The following tests were conducted as evidence that the previously identified adsorbents could remove dialkyl sulfides from an ether synthesis hydrocarbon stream.

The dynamic experiments were carried out in a laboratory-scale, continuous-flow tubular reactor. The adsorbent, generally 5 cm³, was placed in a metal tube and held in place by porous metal plugs. The tube was kept at ambient temperature. The single liquid feed was introduced by an HPLC pump controlling the liquid flow to yield a Liquid Hourly Space Velocity of 4. The back pressure at the exit of the tube was kept at 300 psig. The adsorbent chosen for this dynamic test was sodium-X zeolite. The feed was a synthetic blend of butene-1, isobutylene, dimethyl sulfide, ethyl mercaptan, and n-heptane. The progress of the adsorption was monitored by taking small liquid samples from the tube exit and analyzing them on a capillary gas chromatograph for dimethyl sulfide and ethyl mercaptan concentrations. The dynamic study results are summarized in the following table.

**TABLE III**

| Run | Feed Composition | Observations |
|---|---|---|
| I | 110 ppm DMS in n-heptane | DMS concentration remained below 1 ppm for 72 hours |
| II | 110 ppm DMS in 5% butene-1, 7% isobutylene, and 88% n-heptane | DMS concentration remained below 1 ppm for 72 hours. |
| III | 30 ppm DMS and 110 ppm ethyl mercaptan in 5% butene-1, 7% isobutylene and 88% n-heptane | DMS concentration remained below 1 ppm for 50 hours. |
| IV | The same as in Run III | The Na-X zeolite used in Run III was regenerated with a hot nitrogen purge at 177° - 204°C (350° -400°F). The regenerated adsorbent showed the same DMS removal as in Run III. |

| Run | Feed Composition | Observations |
|---|---|---|
| V-XII | The same as in Run III | 8 cycles of regeneration were conducted. In each cycle the DMS concentration in product was below 1 ppm for 50 hours. |

Related to this, sulfur components in a feed stream have historically presented a problem because of their tendency to deactivate catalysts. Mercaptans can usually be removed by a caustic wash, but this method has not been found to be effective in removing sulfides, such as dialkyl sulfides.

Therefore, it was unexpectedly discovered that metal oxides on various supports can be effective in removing both mercaptans and sulfides. For this purpose metal oxides such as MoO₃, NiO, Cr₂O₃, and CoO and their mixtures have been screened on supports such as alumina and carbon, and were found to be effective to various degrees in reducing the concentration of mercaptans and sulfides present in an olefinic/hydrocarbon streams at room temperature.

### EXAMPLE IV

Static tests were carried out on a group of metal oxides to determine their capability for removing sulfur compounds from a hydrocarbon refinery stream.

These metal oxides were screened at ambient temperature using a synthetically blended feed of ethyl mercaptan, dimethylsulfide and butene-1 in heptane.

Samples were removed for gas chromatographic analysis after 24 hours. Results are listed in the table below.

| | |
|---|---|
| Feed Composition: | 90.1% n-heptane |
| | 9.0 butene-1 |
| | 43 ppm dimethylsulfide |
| | 104 ppm ethylmercaptan |

| Metal Oxide | Product Sulfur Level |
|---|---|
| NiO/MoO₃/Alumina | < 1 ppm Sulfur |
| CoO/MoO₃/Alumina | < 1 ppm Sulfur |
| MoO₃/Carbon | < 1 ppm Sulfur |
| Cr₂O₃/Alumina | no Sulfur |

Thus, not only is the present invention based on the discovery that the presence of dialkyl sulfides in the hydrocarbon stream exposed to the catalyst is responsible for deactivation of the catalyst material, but is also directed to an unexpected procedure for removing this particular sulfur contaminant from the hydrocarbon stream.

### EXAMPLE V

The following tests were conducted to substantiate the previous findings that the presence of dimethyl sulfide reacts with the catalyst so as to cause the catalyst to become deactivated during the preparation of MTBE from isobutylene and methanol over Amberlyst-15 (trademark).

Samples of deactivated catalysts were removed from three locations in a synthesis tower used in the commercial preparation of MTBE for isobutylene and methanol. Each of these samples, along with a sample of fresh unused catalyst, was analyzed on a cross-polarization magic angle spinning (CPMAS) carbon 13 nuclear magnetic resonance (C¹³NMR) instrument.

All three deactivated samples exhibited a sharp peak at 27 ppm, whereas the fresh catalyst did not exhibit such a peak. In this regard, the literature reports a range of 27.5-28.1 ppm for the C¹³NMR of the trimethylsulfonium ion.

To further substantiate the indication that the reaction of dimethyl sulfide with the catalyst causes a deactivation of same, one of the samples of used catalyst was reacted with 10% DCl in D₂O and the resultant solution was used for proton NMR and C¹³NMR. If the trimethyl sulfonium cation were present it would be expected to be hydrolyzed and the resultant solution should exhibit peaks for this cation. The proton NMR showed a peak at 3.13 ppm. The C¹³ NMR gave only one peak at 27.17 ppm. In each instance, these peaks were found as expected.

Finally, fresh catalyst, i.e., Amberlyst-15 (trademark), was reacted with a solution of trimethylsulfonium iodide in water/methanol at room temperature. The resultant solid was carefully washed free of any excess reactants and dried at 100°C in vacuum. The NMR of this material now exhibited the same peak as the used catalyst.

The foregoing findings substantiate the discovery of the present invention, i.e., that dimethyl sulfide reacts with the catalyst thereby depositing trimethylsulfonium cations on the catalyst during catalytic reactions of feedstreams and the preparation of MTBE from isobutylene and methanol over Amberlyst-15 (trademark) catalyst.

## Claims

1. A process for the production of an ether by contacting a hydrocarbon feed stream in a reaction zone of a reactor in the presence of an acid catalyst characterised in that to minimise deactivation of said catalyst resulting from a reaction of dialkyl sulfide with said catalyst, dialkyl sulfide is removed by adsorption from the hydrocarbon feed stream prior to introduction into the reactor.

2. A method according to claim 1 for producing alkyl tertiary alkyl ether wherein the hydrocarbon feed stream comprises an isoolefin and an alcohol.

3. A method according to claim 2 characterised in that an effective amount of an oxygenate is also provided in the reaction zone to inhibit the reaction of dialkyl sulfide with the catalyst.

4. The method according to either of claims 2 or 3 wherein said isoolefin is selected from isobutene and isoamylene, and said alcohol is selected from methanol and ethanol.

5. The method according to any one of claims 1 to 4 wherein said acid catalyst is a cation exchange resin catalyst, preferably a macroreticular sulfonic acid cation exchange resin catalyst.

6. The method according to any one of claims 3 to 5 wherein said oxygenate is an oxygen-containing hydrocarbon selected from ether, alcohol and mixtures thereof.

7. The method as defined by claim 6, wherein said ether is selected from methyl sec-butyl ether, methyl n-butyl ether methyl tert-butyl ether (MTBE), and tert-amyl methyl ether (TAME), and said alcohols are selected from tert-butyl alcohol, C3 alcohols, C4 alcohols, C5 alcohols, ethanol and methanol.

## Patentansprüche

1. Verfahren zur Herstellung eines Ethers durch Kontaktieren eines Kohlenwasserstoffeinsatzmaterialstroms in einer Reaktionszone eines Reaktors in Gegenwart eines Säurekatalysators, dadurch gekennzeichnet, daß zur Minimierung der Desaktivierung des Katalysators, die aus einer Reaktion von Dialkylsulfid mit dem Katalysator resultiert, vor der Einbringung in den Reaktor Dialkylsulfid durch Adsorption aus dem Kohlenwasserstoffeinsatzmaterialstrom entfernt wird.

2. Verfahren nach Anspruch 1 zur Herstellung von Alkyl-tert.-alkylether, bei dem der Kohlenwasserstoffeinsatzmaterialstrom ein Isoolefin und einen Alkohol umfaßt.

3. Verfahren nach Anspruch 2, dadurch gekennzeichnet, daß auch eine wirksame Menge eines Oxygenats (einer sauerstoffhaltigen organischen Verbindung) in der Reaktionszone bereitgestellt wird, um die Reaktion des Dialkylsulfids mit dem Katalysator zu hemmen.

4. Verfahren nach einem der Ansprüche 2 oder 3, bei dem das Isoolefin ausgewählt ist aus Isobuten und Isoamylen und der Alkohol ausgewählt ist aus Methanol und Ethanol.

5. Verfahren nach einem der Ansprüche 1 bis 4, bei dem der Säurekatalysator ein Kationenaustauscherharzkatalysator, vorzugsweise ein makroretikularer Kationenaustauscher-Sulfonsäureharzkatalysator ist.

6. Verfahren nach einem der Ansprüche 3 bis 5, bei dem das Oxygenat ein sauerstoffhaltiger Kohlenwasserstoff ausgewählt aus Ether, Alkohol und Mischungen daraus ist.

7. Verfahren nach Anspruch 6, bei dem der Ether ausgewählt ist aus Methyl-sec-butylether, Methyl-n-butylether, Methyl-tert.-butylether (MTBE) und tert.-Amylmethylether (TAME) und die Alkohole ausgewählt sind aus tert.-Butylalkohol, C₃-Alkoholen, C₄-Alkoholen, C₅-Alkoholen, Ethanol und Methanol.

## Revendications

1. Procédé pour la production d'un éther par mise en contact d'un courant d'hydrocarbures d'alimentation dans une zone réactionnelle d'un réacteur en présence d'un catalyseur acide, caractérisé en ce que, pour réduire au minimum la désactivation dudit catalyseur résultant d'une réaction d'un sulfure de dialkyle avec ledit catalyseur, le sulfure de dialkyle est éliminé par adsorption à partir du courant d'hydrocarbures d'alimentation avant introduction dans le réacteur.

2. Procédé suivant la revendication 1 pour la production d'un éther d'alkyle et tertio-alkyle, dans lequel le courant d'hydrocarbures d'alimentation comprend une iso-oléfine et un alcool.

3. Procédé suivant la revendication 2, caractérisé en ce qu'une quantité efficace d'un produit d'oxygénation est également introduite dans la zone réactionnelle pour inhiber la réaction du sulfure de dialkyle avec le catalyseur.

4. Procédé suivant une des revendications 2 et 3, dans lequel l'iso-oléfine est choisie entre l'isobutylène et l'iso-amylène, et l'alcool est choisi entre le méthanol et l'éthanol.

5. Procédé suivant l'une quelconque des revendications 1 à 4, dans lequel le catalyseur acide est un catalyseur à base d'une résine échangeuse de cations, de préférence un catalyseur à base d'une résine acide sulfonique macroréticulaire échangeuse de cations.

6. Procédé suivant l'une quelconque des revendications 3 à 5, dans lequel le produit d'oxygénation est un hydrocarbure contenant de l'oxygène, choisi entre un éther, un alcool et leurs mélanges.

7. Procédé répondant à la définition suivant la revendication 6, dans lequel l'éther est choisi entre l'éther de méthyle et sec.-butyle, l'éther de méthyle et n-butyle, l'éther de méthyle et tertio-butyle (MTBE), et l'éther de tertio-amyle et de méthyle (TAME), et les alcools sont choisis entre l'alcool tertio-butylique, des alcools en C₃, des alcools en C₄, des alcools en C₅, l'éthanol et le méthanol.
